# FASCICULE DE BREVET EUROPEEN

(11) **EP 0 893 438 B1**
(45) Date de publication et mention de la délivrance du brevet: **10.04.2002**
(21) Numéro de dépôt: 98202332.7
(22) Date de dépôt: 10.07.1998
(51) Int. Cl.: C07C 409/34, C07C 407/00, C08F 4/34, C08F 14/22

(54) **Solution organique de peroxydicarbonate de dialkyle, procédé pour l'obtenir, préparation de polymères halogénés à l'intervention de celle-ci et polymères halogénés obtenus**
Organische Dialkylperoxydicarbonatlösung, Verfahren zu ihrer Herstellung, Herstellung von halogenierten Polymeren unter Verwendung derselben und erhaltene halogenierte Polymere
Organic solution of dialkyl peroxydicarbonate, process for its production, preparation of halogenated polymers using the same and resulting halogenated polymers

(30) Priorité: 22.07.1997 BE 9700633
(43) Date de publication de la demande: 27.01.1999
(73) Titulaire: SOLVAY (Société Anonyme), 1050 Bruxelles (BE)
(72) Inventeur: Lannuzel, Thierry, 39500 Taveaux (FR); Bodart, Vincent, 5001 Namur (BE); Bacque, Xavier, 39500 Taveaux (FR); Laurent, Guy, 5020 Vedrin (BE); Declerck, Fredy, 1850 Grimbergen (BE)
(74) Mandataire: Dufrasne, Eugène

(56) Documents cités:
- GB-A- 1 055 985
- GB-A- 1 484 675
- US-A- 4 524 194
- DATABASE WPI Section Ch, Week 8722 Derwent Publications Ltd., London, GB; Class A14, AN 87-155165 XP002061856 & RO 90 603 A (COMB CHIMIC RIMNICU) , 29 novembre 1986
- PATENT ABSTRACTS OF JAPAN vol. 018, no. 122 (C-1173), 28 février 1994 & JP 05 310915 A (ASAHI CHEM IND CO LTD), 22 novembre 1993

## Description

La présente invention concerne des solutions organiques de peroxydicarbonate de dialkyle et leur utilisation pour la polymérisation de monomères halogénés afin d'obtenir des polymères de propriétés améliorées.

Il est connu de recourir à des peroxydicarbonates de dialkyle pour initier la polymérisation en suspension aqueuse de monomères halogénés. Les peroxydicarbonates de dialkyle constituent des initiateurs particulièrement appréciés du fait de leur activité élevée aux températures usuelles de polymérisation. Mais, ils présentent toutefois l'inconvénient d'être instables, de sorte que leur stockage à l'état pur présente des risques très sérieux de sécurité.

En vue de pallier cet inconvénient, on a déjà proposé de fabriquer ces peroxydicarbonates de dialkyle dans le réacteur de polymérisation ("in-situ"). Ce procédé de préparation "in-situ" ne permet cependant pas une automatisation de l'alimentation en initiateur des réacteurs de polymérisation. En outre, ce procédé manque de reproductibilité en raison du manque de précision sur les quantités d'initiateur effectivement mises en oeuvre à la polymérisation. Ce procédé manque également de productivité car il est nécessaire de faire précéder chaque cycle de polymérisation par la synthèse "in-situ" de l'initiateur. Les sous-produits et résidus de la synthèse du peroxydicarbonate de dialkyle ne sont par ailleurs pas éliminés.

On a également déjà proposé de préparer la quantité juste nécessaire de peroxydicarbonate de dialkyle en dehors du réacteur de polymérisation ("ex-situ") et immédiatement avant la polymérisation. Cette préparation s'effectue par réaction d'un halogénoformiate d'alkyle avec un composé peroxydé en présence d'eau et d'un solvant volatil non miscible à l'eau ayant, de préférence une température d'ébullition inférieure à 100 °C. L'ensemble du milieu réactionnel dans lequel le peroxydicarbonate de dialkyle a été préparé (phase aqueuse et phase organique) est alors introduit dans le réacteur de polymérisation lequel est ensuite chargé en vue de la polymérisation (brevet belge 822913 au nom de SOLVAY et Cie). Le solvant volatil est de préférence éliminé, en tout ou en partie, avant la polymérisation par mise sous vide.

Ce procédé permet l'automatisation de l'alimentation en initiateur des réacteurs de polymérisation mais nécessite toujours de produire la quantité juste suffisante d'initiateur immédiatement avant la polymérisation. Une introduction en différé de l'initiateur qui est une technique intéressante par exemple pour améliorer la cinétique de la polymérisation, n'est dès lors pas réalisable. Ce procédé ne permet par ailleurs pas de disposer d'une solution de peroxydicarbonate de dialkyle qui puisse être stockée en toute sécurité et utilisable à tout moment. De plus, les impuretés hydrosolubles présentes dans la phase aqueuse après la préparation du peroxydicarbonate de dialkyle ne sont pas éliminées avant l'introduction dans le réacteur de polymérisation.

On a enfin déjà proposé de polymériser le fluorure de vinylidène en suspension aqueuse à l'intervention d'un initiateur radicalaire oléosoluble et d'un agent régulateur de chaînes choisi parmi les carbonates de dialkyle contenant au plus 5 atomes de carbone, afin d'améliorer la stabilité thermique du polymère (brevet US 4524194 au nom de SOLVAY et Cie). Toutefois, le brevet précité ne divulgue pas de solution d'initiateur radicalaire dans un carbonate de dialkyle.

Afin de remédier aux inconvénients présentés par les procédés de l'art antérieur, la présente invention a pour objet de procurer une solution organique de peroxydicarbonate de dialkyle, et plus particulièrement de peroxydicarbonate de diéthyle, qui est particulièrement adaptée pour la préparation de polymères halogénés, et plus particulièrement de polymères contenant du fluor (incluant les polymères du fluorure de vinylidène) par polymérisation en suspension aqueuse, ainsi qu'un procédé perfectionné pour la fabriquer.

L'invention a également pour objet un procédé simple et efficace de préparation de polymères halogénés, et plus particulièrement de polymères contenant du fluor (incluant les polymères du fluorure de vinylidène) par polymérisation en suspension aqueuse à l'intervention de cette solution organique.

L'invention a également pour objet les polymères halogénés, et plus particulièrement les polymères contenant du fluor (incluant les polymères du fluorure de vinylidène) ainsi obtenus.

La présente invention concerne tout d'abord un procédé perfectionné de préparation d'une solution organique de peroxydicarbonate de dialkyle qui est particulièrement adaptée pour être mise en oeuvre à la polymérisation en suspension aqueuse de monomères halogénés.

A cet effet, l'invention concerne un procédé de préparation d'une solution organique de peroxydicarbonate de dialkyle selon lequel on met en réaction dans l'eau un halogénoformiate d'alkyle en quantités appropriées avec un peroxyde inorganique et on sépare le peroxydicarbonate de dialkyle obtenu par extraction au moyen d'un solvant organique insoluble dans l'eau choisi parmi les carbonates de dialkyle afin d'obtenir une solution de peroxydicarbonate de dialkyle dans ce solvant.

Le peroxydicarbonate de dialkyle est de préférence le peroxydicarbonate de diéthyle.

L'halogénoformiate d'alkyle est le plus souvent et avantageusement un chloroformiate d'alkyle. Le peroxyde inorganique est le plus souvent du peroxyde de calcium ou de sodium ou encore de l'eau oxygénée. Dans ce dernier cas, il convient d'introduire en outre dans le milieu aqueux de réaction une base, telle que l'hydroxyde de calcium ou encore l'hydroxyde de sodium. De manière préférée, le peroxyde inorganique est l'eau oxygénée et de l'hydroxyde de sodium est alors ajouté au milieu réactionnel.

La quantité d'eau oxygénée est habituellement inférieure ou égale à la quantité stoechiométrique. Elle est en général supérieure ou égale à un défaut stoechiométrique de 5 % par rapport à la quantité d'halogénoformiate d'alkyle. La quantité d'hydroxyde de sodium est habituellement inférieure ou égale à la quantité stoechiométrique. Elle est en général supérieure ou égale à un défaut stoechiométrique de 5 % par rapport à la quantité d'halogénoformiate d'alkyle. Le défaut stoechiométrique ne doit pas nécessairement être le même pour l'eau oxygénée et l'hydroxyde de sodium. Un défaut stoechiométrique de 3 % pour l'hydroxyde de sodium et de 4 % pour l'eau oxygénée par rapport à la quantité d'halogénioformiate d'alkyle, donne habituellement de bons résultats.

La réaction entre l'halogénioformiate d'alkyle, le peroxyde d'hydrogène et l'hydroxyde de sodium s'effectue habituellement sous vive agitation. La température de la réaction est le plus souvent maintenue à une valeur située entre -5 °C et + 15 °C, de préférence entre 0 °C et + 15 °C. La durée totale de la préparation du peroxydicarbonate de dialkyle est réglée par la durée de l'addition de l'hydroxyde de sodium au milieu aqueux contenant l'halogénioformiate d'alkyle et le peroxyde d'hydrogène qui varie habituellement de quelques dizaines de minutes à quelques heures.

La séparation par extraction du peroxydicarbonate de dialkyle obtenu s'effectue de toute manière connue et appropriée. Avantageusement, on ajoute le solvant d'extraction au milieu de réaction aqueux de préparation du peroxydicarbonate de dialkyle sous vive agitation, on laisse ensuite décanter les phases après avoir arrêté l'agitation et on sépare la phase organique de la phase aqueuse pour recueillir une solution pure du peroxydicarbonate de dialkyle dans le solvant d'extraction.

Le solvant d'extraction peut être ajouté au milieu de réaction aqueux à tout moment de la réaction de formation du peroxydicarbonate de dialkyle c'est-à-dire du moment allant de l'introduction des principaux réactifs jusqu'après la synthèse du peroxydicarbonate de dialkyle. Par ailleurs, le solvant d'extraction peut être ajouté en une seule fois ou en plusieurs fois.

La quantité de solvant utilisée pour l'extraction n'est pas critique. Il va de soi qu'elle dépendra notamment du degré de solubilité du peroxydicarbonate de dialkyle dans le solvant choisi. Avantageusement, cette quantité sera telle que la concentration finale en peroxydicarbonate de dialkyle de la solution organique s'élève d'environ 15 à 40 % en poids et plus particulièrement d'environ 20 à 35 % en poids.

Dans le cas où la densité de la phase aqueuse finale après formation du peroxydicarbonate de dialkyle est inférieure à 1,05, il est alors nécessaire de densifier la phase aqueuse par l'ajout d'un sel inorganique soluble dans l'eau en quantité suffisante pour augmenter la densité du milieu aqueux de réaction et faciliter dès lors la décantation et la séparation des phases aqueuse et organique.

On met alors en oeuvre le sel inorganique en quantité suffisante pour amener la densité du milieu aqueux de réaction à une valeur supérieure à la densité de la solution organique réalisée dans la deuxième étape. La densité de la phase aqueuse est de préférence au moins égale à 1,05 et plus particulièrement encore à une valeur au moins égale à 1,06. Par ailleurs, il convient d'adapter la quantité de sel inorganique de telle sorte qu'elle ne dépasse pas la concentration de saturation en sel du milieu aqueux de réaction.

La nature du sel mis en oeuvre au stade de la préparation du peroxydicarbonate de dialkyle n'est pas particulièrement critique. En principe, tout sel inorganique qui n'interfère pas avec la réaction de formation du peroxydicarbonate de dialkyle et qui ne précipite pas dans les conditions de réaction convient. A titre d'exemples non limitatifs de tels sels, on peut citer par exemple les halogénures et en particulier les chlorures des métaux alcalins et alcalino-terreux, en particulier le chlorure de sodium mais également les sulfates de métaux alcalins et alcalino-terreux tel le sulfate de sodium ou les nitrates de métaux alcalins et alcalino-terreux tel le nitrate de calcium.

La présente invention concerne également une solution organique de peroxydicarbonate de dialkyle dans un solvant organique insoluble dans l'eau particulièrement adaptée pour être mise en oeuvre à la polymérisation en suspension aqueuse de monomères halogénés.

A cet effet, l'invention concerne une solution organique de peroxydicarbonate de dialkyle dans un solvant organique insoluble dans l'eau selon laquelle ce solvant est choisi parmi les carbonates de dialkyle.

Le peroxydicarbonate de dialkyle est de préférence le peroxydicarbonate de diéthyle.

La présente invention concerne en outre une solution organique de peroxydicarbonate de dialkyle, obtenue par le procédé de préparation faisant l'objet de l'invention.

La concentration en peroxydicarbonate de dialkyle dans la solution organique selon l'invention s'élève généralement d'environ 15 à 40 % en poids. De préférence, la concentration en peroxydicarbonate de dialkyle dans la solution organique s'élève d'environ 20 à 35 % en poids.

L'invention concerne également un procédé pour la préparation de polymères halogénés par polymérisation en suspension aqueuse de monomères halogénés à l'intervention de peroxydicarbonate de dialkyle, selon lequel le peroxydicarbonate de dialkyle est mis en oeuvre à la polymérisation sous la forme d'une solution organique dans un solvant organique insoluble dans l'eau choisi parmi les carbonates de dialkyle.

Le procédé de préparation de polymères halogénés s'applique de préférence à la préparation de polymères contenant du fluor et de manière plus que préférée à la préparation de polymères du fluorure de vinylidène.

Le peroxydicarbonate de dialkyle est de préférence le peroxydicarbonate de diéthyle.

La concentration en peroxydicarbonate de dialkyle des solutions mises en oeuvre dans le procédé de préparation de polymères halogénés selon l'invention s'élève généralement d'environ 15 à 40 % en poids. De bons résultats sont obtenus avec des solutions dont la concentration en peroxydicarbonate de dialkyle s'élève d'environ 20 à 35 % en poids.

La solution organique de peroxydicarbonate de dialkyle selon l'invention est mise en oeuvre en des quantités telles que le peroxydicarbonate de dialkyle soit présent dans le milieu de polymérisation en des quantités habituelles. Les quantités usuelles du peroxydicarbonate de dialkyle sont d'environ 0,05 à 3 % en poids par rapport au monomère mis en oeuvre et, de préférence d'environ 0,05 à 2 % en poids. Les quantités de l'agent régulateur de chaînes, en l'occurrence de carbonate de dialkyle, mises en oeuvre à la polymérisation sont usuellement d'environ 0,5 à 5 % en poids par rapport au monomère mis en oeuvre. Il peut être nécessaire d'ajouter des quantités supplémentaires de l'agent régulateur de chaînes, le carbonate de dialkyle, en plus de celles mises en oeuvre par l'introduction de la solution organique de peroxydicarbonate de dialkyle dans l'agent de transfert carbonate de dialkyle.

Habituellement, les peroxydicarbonates de dialkyle en solution organique sont introduits au début de la polymérisation. Il est cependant entendu que les peroxydicarbonates de dialkyle en solution organique peuvent être introduits, en tout ou en partie après le début de la polymérisation (en différé). La mise en oeuvre en différé d'une partie du peroxydicarbonate de dialkyle peut être avantageuse pour améliorer la cinétique de la polymérisation.

Mise à part la particularité de la mise en oeuvre d'un peroxydicarbonate de dialkyle sous la forme d'une solution organique dans un solvant organique insoluble dans l'eau selon l'invention, les conditions générales de la polymérisation suivant le procédé de l'invention ne different pas de celles habituellement mises en oeuvre pour la préparation de polymères halogénés, plus particulièrement de polymères contenant du fluor et en particulier de polymères du fluorure de vinylidène, par polymérisation en suspension aqueuse de monomères halogénés.

Par polymérisation en suspension aqueuse, on entend la polymérisation à l'intervention d'initiateurs oléosolubles, en l'occurrence notamment des peroxydicarbonates de dialkyle et en présence d'agent dispersant.

Dans le cas particulier de la préparation de polymères du fluorure de vinylidène, les agents dispersants sont habituellement des dérivés cellulosiques hydrosolubles, tels que les alkyl- et les alkylhydroxyalkylcelluloses. La quantité d'agent dispersant mise en oeuvre varie généralement entre 0,01 et 0,5 % en poids par rapport au(x) monomère(s) mis en oeuvre. Les meilleurs résultats sont obtenus lorsqu'on en utilise de 0,02 à 0,2 % en poids.

La température de polymérisation peut se situer indifféremment en dessous ou au-dessus de la température critique du fluorure de vinylidène (30,1 °C). Lorsque la température se situe en dessous de 30,1 °C, la polymérisation s'effectue au sein d'une suspension aqueuse classique de fluorure de vinylidène liquide sous une pression égale à la pression de vapeur saturante du fluorure de vinylidène. Lorsque la température se situe au-dessus de 30,1 °C, elle s'effectue au sein d'une suspension aqueuse de fluorure de vinylidène gazeux se trouvant avantageusement sous pression élevée. On peut donc réaliser le procédé selon l'invention à des températures allant de la température ambiante à environ 110 °C. On préfère néanmoins effectuer la polymérisation à une température située au-dessus de 30,1 °C. Suivant un mode de réalisation préféré du procédé selon l'invention, on effectue la polymérisation du fluorure de vinylidène à une température comprise entre 35 et 100 °C et sous des pressions initiales d'environ 55 à 200 bars. On peut, bien entendu, augmenter la productivité des réacteurs en procédant en cours de polymérisation à des injections supplémentaires de monomère ou d'eau ou à une élévation de la température de polymérisation.

La polymérisation est en général réalisée en discontinu. Elle est en général réalisée dans des réacteurs à cuve pourvus d'un agitateur à pales, sabres ou turbine.

En fin de polymérisation, les polymères du fluorure de vinylidène obtenus selon le procédé de l'invention sont isolés de manière conventionnelle de leur milieu de polymérisation, par essorage suivi de séchage.

L'invention concerne également les polymères halogénés obtenus par le procédé selon l'invention. Ceux-ci sont caractérisés en ce qu'ils ont au moins l'une des propriétés suivantes qui est améliorée : a) stabilité thermique, b) pureté.

Par stabilité thermique améliorée, on entend que l'indice de jaune YI, mesuré suite au test BOY et/ou l'indice de jaune YI (10 min) mesuré suite au test BOY (10 min) sont/est amélioré(s).

Par pureté améliorée, on entend que le polymère halogéné se caractérise par une teneur réduite en ions métalliques.

Les polymères halogénés sont de préférence des polymères contenant du fluor et de manière plus que préférée, des polymères du fluorure de vinylidène.

Les termes utilisés dans le présent texte sont définis ci-après.

Par solution organique de peroxydicarbonate de dialkyle dans un solvant organique insoluble dans l'eau, on entend désigner, aux fins de la présente invention, que la solution organique est constituée essentiellement du peroxydicarbonate de dialkyle et du solvant organique insoluble dans l'eau. Elle est donc exempte de toute phase aqueuse provenant du milieu réactionnel dans lequel le peroxydicarbonate de dialkyle a été préparé.

Par peroxydicarbonate de dialkyle, on entend désigner, aux fins de la présente invention, les peroxydicarbonates dont les radicaux alkyles contiennent au moins 2 atomes de carbone et représentent les radicaux éthyle, n-propyle, isopropyle, n-butyle, iso-butyle, tert-butyle, n-octyle, 2-éthylhexyle, cyclohexyle, 4-tert-butylcyclohexyle, myristyle ou cétyle. Parmi ceux-ci, on donne la préférence aux peroxydicarbonates de diéthyle et de diisopropyle. Un peroxydicarbonate de dialkyle tout particulièrement préféré est le peroxydicarbonate de diéthyle.

Par solvant organique insoluble dans l'eau, on entend désigner, aux fins de la présente invention, les solvants organiques liquides et insolubles dans l'eau dans les conditions normales, c'est-à-dire à température ambiante et sous pression atmosphérique.

Par insoluble dans l'eau, on entend plus particulièrement une solubilité dans l'eau à température ambiante inférieure à 15 % en poids. De préférence, la solubilité dans l'eau des carbonates de dialkyle servant de solvant pour le peroxydicarbonate de dialkyle dans le procédé de l'invention, ne dépasse pas 5 % en poids et de manière plus que préférée, elle ne dépasse pas 3 % en poids.

Les carbonates de dialkyle les plus efficaces, auxquels on donne par conséquent la préférence, sont ceux dont les groupements alkyles contiennent cinq atomes de carbone au plus. A titre d'exemples de pareils carbonates de dialkyle, on peut citer les carbonates de diméthyle, de diéthyle, de di(n-propyle), de di(n-butyle), de di(sec-butyle), de di(iso-butyle), de di(tert-butyle), de di(n-pentyle), de di(iso-amyle) et de di(néo-pentyle).

Un carbonate de dialkyle tout particulièrement préféré selon l'invention est le carbonate de diéthyle (solubilité dans l'eau à température ambiante : 1,9 % en poids, densité : 0,975).

Par polymères halogénés, on entend désigner aux fins de la présente invention, aussi bien les homopolymères que les copolymères de monomères halogénés; notamment les homopolymères de monomères halogénés tels que le fluorure de vinylidène, le fluorure de vinyle, le trifluoréthylène, le tétrafluoréthylène, le chlorotrifluoréthylène, l'hexafluoropropylène, le chlorure de vinyle, le chlorure de vinylidène ainsi que les copolymères de ces monomères halogénés et les copolymères d'un de ces monomères halogénés avec un autre monomère à insaturation éthylénique tel l'éthylène, les monomères acryliques ou méthacryliques, l'acétate de vinyle.

Par polymères contenant du fluor, on entend désigner aux fins de la présente invention, aussi bien les homopolymères que les copolymères de monomères contenant du fluor, notamment les homopolymères du fluorure de vinylidène, du fluorure de vinyle, du trifluoréthylène, du tétrafluoréthylène, du chlorotrifluoréthylène, de l'hexafluoropropylène ainsi que les copolymères de ces monomères contenant du fluor tels que par exemple le copolymère du tétrafluoréthylène et de l'hexafluoropropylène, les copolymères du fluorure de vinylidène avec un autre monomère fluoré tel que défini ci-dessus et les copolymères du fluorure de vinyle avec un autre monomère fluoré tel que défini ci-dessus. Les copolymères d'un des monomères contenant du fluor cités ci-dessus avec un autre monomère à insaturation éthylénique sont également considérés. Le copolymère du tétrafluoréthylène et de l'éthylène et le copolymère du trifluoréthylène et de l'éthylène en sont des exemples.

Par polymères du fluorure de vinylidène, on entend désigner aux fins de la présente invention aussi bien les homopolymères du fluorure de vinylidène, que ses copolymères avec d'autres monomères à insaturation eylénique, avantageusement fluorés . A titre d'exemples de comonomères fluorés utilisables, on peut mentionner le fluorure de vinyle, le trifluoréthylène, le chlorotrifluoréthylène, le tétrafluoréthylène et l'hexafluoropropylène. Les copolymères obtenus contiennent de préférence au moins environ 75 % en poids d'unités monomériques dérivées du fluorure de vinylidène. Avantageusement lesdits copolymères thermoplastiques présentent une température de fusion au moins égale à 130 °C et, de préférence, au moins égale à 150 °C et plus particulièrement encore à 165 °C.

Le procédé de préparation de solutions organiques de peroxydicarbonates de dialkyle selon l'invention procure des solutions présentant de nombreux avantages. En effet, étant donné que les impuretés apparaissant lors de la préparation sont hydrosolubles et éliminées avec la phase aqueuse, les solutions obtenues avec un rendement élevé sont très pures. Les solutions de peroxydicarbonates de dialkyle obtenues par le procédé selon l'invention peuvent être stockées sans inconvénient pendant des laps de temps relativement longs (plusieurs mois) sans perte d'activité notable. Le stockage s'effectue généralement à basse température, de préférence à une température inférieure à moins 10 °C, de préférence aux environs de moins 20 °C. Des quantités relativement importantes de la solution organique de peroxydicarbonate de dialkyle, suffisantes pour un grand nombre de cycles de polymérisation peuvent donc être préparées et stockées ensuite pour être utilisées au fur et à mesure des besoins. Ces solutions sont prêtes à l'emploi et ne nécessite pas l'élimination préalable du solvant qui a un rôle à jouer lors de la polymérisation. Une même solution peut servir à alimenter plusieurs réacteurs de polymérisation. Enfin, les solutions obtenues peuvent être véhiculées et ne conduisent pas à des problèmes de dépôts dans les conduits.

Le procédé de préparation de polymères halogénés selon l'invention présente de multiples avantages. En particulier, il permet une automatisation de l'alimentation des réacteurs. Il conduit à une amélioration de la reproductibilité des cycles de polymérisation. Il permet également une augmentation de la productivité. Le procédé de polymérisation de l'invention présente également l'avantage de faire intervenir une solution du peroxydicarbonate de dialkyle dans un solvant qui est lui-même partie prenante à la polymérisation. Par ailleurs, la mise en oeuvre des peroxydicarbonates de dialkyle sous la forme d'une solution organique selon l'invention permet d'obtenir des résines présentant soit une meilleure stabilité thermique, soit une pureté accrue ou les deux, par rapport aux résines obtenues en préparant le peroxydicarbonate de dialkyle "in-situ".

### Exemple 1 - Préparation d'une solution de peroxydicarbonate de dialkyle

Dans un autoclave de 4 litres préalablement mis sous vide, on introduit 1300 g d'eau déminéralisée. Après avoir régulé la température de l'autoclave à + 3 °C, on introduit ensuite successivement dans la solution aqueuse agitée au moyen d'un agitateur à sabres (vitesse de 400 tours/minute), 111,7 g d'eau oxygénée à 70 % et 600 g de chloroformiate d'éthyle préalablement refroidi entre 0 et 5 °C alors que l'autoclave est toujours sous vide. La tuyauterie amenant le chloroformiate d'éthyle est alors rincée avec 500 g d'eau déminéralisée et la vitesse d'agitation est augmentée à 750 tours/minute. L'autoclave est ensuite mis à pression atmosphérique avant d'introduire lentement 780 ml d'une solution d'hydroxyde de sodium 6 N en 1 heure. La température est régulée à +3 °C ± 2 °C pendant toute la durée de l'introduction de l'hydroxyde de sodium. Après la fin de l'introduction de l'hydroxyde de sodium, le milieu réactionnel est maintenu sous agitation pendant 10 minutes. La température de l'autoclave est ensuite indexée à + 1 °C et, sous une vitesse d'agitation réduite à 200 tours/minute, on introduit 1300 g de carbonate de diéthyle préalablement refroidi entre 0 et 5 °C. Après avoir maintenu le milieu réactionnel sous agitation à 750 tours/minute pendant 10 minutes, on arrête alors l'agitation et on laisse décanter pendant 30 minutes. On remet ensuite l'autoclave à la pression atmosphérique et on soutire les phases organique et aqueuse. On recueille ainsi 1631 g de solution organique ayant un titre de 245 g/kg en peroxydicarbonate de diéthyle, soit un rendement de 85 % par rapport au peroxyde d'hydrogène. La solution de peroxydicarbonate de diéthyle ainsi produite est stockée au froid (environ moins 20 °C) en vue de son utilisation ultérieure.

### Exemple 2 - Préparation d'un polymère halogéné

Dans un réacteur de 30,8 litres, agité vigoureusement et muni d'une double enveloppe, on introduit successivement 19 kg d'eau déminéralisée et 270 g d'une solution aqueuse d'éthylhydroxypropylcellulose à 15 g/kg. On élimine la plus grande partie de l'oxygène présent dans le réacteur par trois mises sous vide à 40 mbars (à 15 °C) avec, après les deux premières mises sous vide, remise sous pression de 1 bar d'azote. On introduit alors sous vide 82 g d'une solution à 24,5 % en poids de peroxydicarbonate de diéthyle dans le carbonate de diéthyle. On ajoute un complément de 131 g de carbonate de diéthyle pur. On introduit ensuite une charge unique de 8 kg de fluorure de vinylidène, puis on met progressivement en chauffe le réacteur jusqu'à atteindre un premier palier de température de 42 °C, d'une durée d'environ 1 heure 30 minutes. On porte ensuite la température à 61 °C et on l'y maintient pendant environ 1 heure 45 minutes. En fin de polymérisation, on dégaze la suspension aqueuse (en abaissant la pression jusqu'à la pression atmosphérique). Le polymère recueilli est lavé et séché jusqu'à poids constant. La durée totale de la polymérisation est de 3 heures 50 minutes. Le taux de conversion mesuré est de 94 %.

### Exemple 3 - Propriétés du polymère halogéné obtenu à l'exemple 2

Le MFI (Melt Flow Index) du polymère obtenu, mesuré selon la norme ISO 1133 à une température de 230 °C et sous un poids de 2,16 kg, est de 12 g/10 minutes. L'indice de jaune YI mesuré suite au test BOY et l'indice de jaune YI (10 min) mesuré suite au test BOY 10 min sont respectivement de 11 et 20. Le test BOY et la méthode de mesure pour la détermination de l'indice de jaune YI sont décrits ci-dessous. La teneur en ions métalliques, et en particulier la teneur en ions sodium est inférieure à 1 mg/kg de poly(fluorure de vinylidène). La méthode de détermination de la teneur en ions métalliques est décrite ci-dessous.

### Description du test de stabilité thermique (Test BOY)

Le poly(fluorure de vinylidène) est soumis à une contrainte thermique et mécanique lors de l'injection d'une éprouvette avec une presse à injecter. La pièce à injecter, de dimension 60x40x4 mm, est injectée par voile (31,5x0,8 mm). Le moule est raccordé à un système de régulation thermique. La température du moule est régulée à 60 ± 2 °C. La presse à injecter utilisée est une presse à injecter BOY 15 S qui se caractérise par une force de verrouillage de 22 tonnes, un cylindre de 22 mm de diamètre, une buse ouverte de 2,1 mm de diamètre comme nez d'injection, un clapet antiretour constitué de 3 canaux en V, une vis caractérisée par un rapport L/D de 20, 3 zones (12D/4D/4D) et un taux de compression de 1,9. Deux résistances chauffantes indépendantes sont placées sur le fourreau (zones 1 et 2) et deux résistances couplées sur la zone du clapet antiretour et le nez d'injection (zone 3). Les températures de ces 3 zones de chauffe sont dans l'ordre 190 °C, 220 °C et 265 °C. Le temps de résidence dans le fourreau est de l'ordre de 5 minutes. L'effet thermique peut être augmenté par le maintien du polymère fondu chaud dans le fourreau de la machine en interrompant le cycle d'injection pendant 10 minutes (test BOY 10 min).

### Description de la méthode de mesure pour la détermination de l'indice de jaune YI

La détermination de l'indice de jaune YI des échantillons est effectuée i selon la norme ASTM D-1925 au moyen d'un spectrocolorimètre "ULTRASCAN" de HUNTERLAB calibré régulièrement grâce à 3 étalons colorimétriques de calibrage HUNTERLAB (blanc, noir, gris). Le référentiel de mesure est le CIELAB 1976, l'illuminant est le D 65 de la CIE, la bande spectrale analysée est de 400 à 700 nm, l'intervalle spectral de mesure est de 5 nm, le diamètre de la plage examinée est de 0,95 cm, l'observateur standard est à 10° et l'observation se fait sous un angle de 8°.

### Description de la méthode de détermination de la teneur en ions métalliques

La teneur en ions métalliques, en particulier la teneur en ions sodium, exprimée en mg/kg de poly(fluorure de vinylidène), est mesurée par spectrométrie d'absorption atomique dans une flamme air-acétylène (F-AAS) à une longueur d'onde de 589,0 nm. Avant l'analyse, l'échantillon est d'abord soumis à une minéralisation par de l'acide sulfurique dans une capsule en platine, suivie d'une calcination à 580 °C avec reprise des cendres par de l'acide chlorhydrique 5 mole/litre et mise au volume en présence de césium (tampon spectral).

### Exemple 4 (comparatif) - Propriétés d'un polymère halogéné obtenu à l'intervention d'un peroxydicarbonate de dialkyle préparé "in-situ"

A titre comparatif, on a reproduit la polymérisation du fluorure de vinylidène dans les mêmes conditions qu'à l'exemple 2, si ce n'est qu'on synthétise d'abord la quantité appropriée de peroxydicarbonate de diéthyle "in-situ" dans le réacteur de polymérisation. Ainsi, dans un réacteur de 30,8 litres, agité vigoureusement et muni d'une double enveloppe, on introduit successivement 19 kg d'eau déminéralisée, 9 g d'hydroxyde de sodium, 38,3 g d'eau oxygénée à 10 % et 270 g d'une solution aqueuse d'éthylhydroxypropylcellulose à 15 g/kg. Après désaération de l'autoclave par mise sous vide et remise sous pression d'azote, on ajoute 24,5 g de chloroformiate d'éthyle et 190 g de carbonate de diéthyle en tant qu'agent régulateur de chaînes. On introduit ensuite une charge unique de 8 kg de fluorure de vinylidène, puis on met progressivement en chauffe le réacteur jusqu'à atteindre un premier palier de température de 42 °C, d'une durée d'environ 2 heures. On porte ensuite la température à 61 °C et on l'y maintient pendant environ 2 heures. En fin de polymérisation, on dégaze la suspension aqueuse (en abaissant la pression jusqu'à la pression atmosphérique). Le polymère recueilli est lavé et séché jusqu'à poids constant. La durée totale de la polymérisation est de 4 heures 40 minutes. Le taux de conversion mesuré est de 94 %.

Le MFI (Melt Flow Index) du polymère obtenu à l'exemple 4, mesuré selon la norme ISO 1133 à une température de 230 °C et sous un poids de 2,16 kg, est de 10 g/10 minutes. L'indice de jaune YI mesuré suite au test BOY et l'indice de jaune YI (10 min) mesuré suite au test BOY 10 min sont respectivement de 13 et 29. La teneur en ions métalliques et en particulier la teneur en ions sodium est de 2 mg/kg de poly(fluorure de vinylidène).

De la comparaison des résultats, il apparaît que la mise en oeuvre du peroxydicarbonate de diéthyle en solution dans le carbonate de diéthyle (selon l'invention) à la polymérisation du fluorure de vinylidène donne lieu à la préparation de polymères du fluorure de vinylidène qui sont significativement plus stables thermiquement et d'une pureté ionique plus élevée (exemples 2 et 3), que ceux obtenus à l'intervention de peroxydicarbonate de diéthyle préparé "in-situ" (exemple 4 comparatif).

## Revendications

1. Procédé de préparation d'une solution organique de peroxydicarbonate de dialkyle, **caractérisé en ce qu'**on met en réaction dans l'eau un halogénoformiate d'alkyle en quantités appropriées avec un peroxyde inorganique et on sépare le peroxydicarbonate de dialkyle obtenu par extraction au moyen d'un solvant organique insoluble dans l'eau choisi parmi les carbonates de dialkyle afin d'obtenir une solution de peroxydicarbonate de dialkyle dans ce solvant.

2. Procédé de préparation d'une solution organique de peroxydicarbonate de dialkyle selon la revendication 1, **caractérisé en ce que** le solvant organique insoluble dans l'eau est choisi parmi les carbonates de dialkyle dont les groupements alkyles contiennent cinq atomes de carbone au plus.

3. Procédé de préparation d'une solution organique de peroxydicarbonate de dialkyle selon l'une quelconque des revendications 1 à 2, **caractérisé en ce que** le solvant organique insoluble dans l'eau est le carbonate de diéthyle.

4. Procédé de préparation d'une solution organique de peroxydicarbonate de dialkyle selon l'une quelconque des revendications 1 à 3, **caractérisé en ce que** le peroxydicarbonate de dialkyle est le peroxydicarbonate de diéthyle.

5. Solution organique de peroxydicarbonate de dialkyle dans un solvant organique insoluble dans l'eau **caractérisée en ce que** ce solvant est choisi parmi les carbonates de dialkyle.

6. Solution organique de peroxydicarbonate de dialkyle selon la revendication 5, **caractérisée en ce que** le solvant organique insoluble dans l'eau est choisi parmi les carbonates de dialkyle dont les groupements alkyles contiennent cinq atomes de carbone au plus.

7. Solution organique de peroxydicarbonate de dialkyle selon l'une quelconque des revendications 5 à 6, **caractérisée en ce que** le solvant organique insoluble dans l'eau est le carbonate de diéthyle.

8. Solution organique de peroxydicarbonate de dialkyle selon l'une quelconque des revendications 5 à 7, **caractérisée en ce que** le peroxydicarbonate de dialkyle est le peroxydicarbonate de diéthyle.

9. Procédé de préparation de polymères halogénés par polymérisation en suspension aqueuse de monomères halogénés à l'intervention de peroxydicarbonate de dialkyle, **caractérisé en ce que** le peroxydicarbonate de dialkyle est mis en oeuvre à la polymérisation sous la forme d'une solution organique dans un solvant organique insoluble dans l'eau choisi parmi les carbonates de dialkyle.

10. Procédé de préparation de polymères halogénés par polymérisation en suspension aqueuse de monomères halogénés selon la revendication 9, **caractérisé en ce que** les polymères halogénés sont des polymères contenant du fluor.

11. Procédé de préparation de polymères halogénés par polymérisation en suspension aqueuse de monomères halogénés selon l'une quelconque des revendications 9 à 10, **caractérisé en ce que** les polymères halogénés sont des polymères du fluorure de vinylidène.

12. Procédé de préparation de polymères halogénés par polymérisation en suspension aqueuse de monomères halogénés selon l'une quelconque des revendications 9 à 11, **caractérisé en ce que** le solvant organique insoluble dans l'eau est choisi parmi les carbonates de dialkyle dont les groupements alkyles contiennent cinq atomes de carbone au plus.

13. Procédé de préparation de polymères halogénés par polymérisation en suspension aqueuse de monomères halogénés selon l'une quelconque des revendications 9 à 12, **caractérisé en ce que** le solvant organique insoluble dans l'eau est le carbonate de diéthyle.

14. Procédé de préparation de polymères halogénés par polymérisation en suspension aqueuse de monomères halogénés selon l'une quelconque des revendications 9 à 13, **caractérisé en ce que** le peroxydicarbonate de dialkyle est le peroxydicarbonate de diéthyle.

## Claims

1. Process for the preparation of an organic solution of dialkyl peroxydicarbonate, **characterized in that** an alkyl haloformate is reacted, in water, in appropriate amounts with an inorganic peroxide and the dialkyl peroxydicarbonate obtained is separated by extraction by means of a water-insoluble organic solvent chosen from dialkyl carbonates, in order to obtain a solution of dialkyl peroxydicarbonate in this solvent.

2. Process for the preparation of an organic solution of dialkyl peroxydicarbonate according to Claim 1, **characterized in that** the water-insoluble organic solvent is chosen from dialkyl carbonates in which the alkyl groups comprise at most five carbon atoms.

3. Process for the preparation of an organic solution of dialkyl peroxydicarbonate according to any one of Claims 1 to 2, **characterized in that** the water-insoluble organic solvent is diethyl carbonate.

4. Process for the preparation of an organic solution of dialkyl peroxydicarbonate according to any one of Claims 1 to 3, **characterized in that** the dialkyl peroxydicarbonate is diethyl peroxydicarbonate.

5. Organic solution of dialkyl peroxydicarbonate in a water-insoluble organic solvent, **characterized in that** this solvent is chosen from dialkyl carbonates.

6. Organic solution of dialkyl peroxydicarbonate according to Claim 5, **characterized in that** the water-insoluble organic solvent is chosen from dialkyl carbonates in which the alkyl groups comprise at most five carbon atoms.

7. Organic solution of dialkyl peroxydicarbonate according to any one of Claims 5 to 6, **characterized in that** the water-insoluble organic solvent is diethyl carbonate.

8. Organic solution of dialkyl peroxydicarbonate according to any one of Claims 5 to 7, **characterized in that** the dialkyl peroxydicarbonate is diethyl peroxydicarbonate.

9. Process for the preparation of halogenated polymers by aqueous suspension polymerization of halogenated monomers with the involvement of dialkyl peroxydicarbonate, **characterized in that** the dialkyl peroxydicarbonate is employed in the polymerization in the form of an organic solution in a water-insoluble organic solvent chosen from dialkyl carbonates.

10. Process for the preparation of halogenated polymers by aqueous suspension polymerization of halogenated monomers according to Claim 9, **characterized in that** the halogenated polymers are polymers comprising fluorine.

11. Process for the preparation of halogenated polymers by aqueous suspension polymerization of halogenated monomers according to either one of Claims 9 and 10, **characterized in that** the halogenated polymers are vinylidene fluoride polymers.

12. Process for the preparation of halogenated polymers by aqueous suspension polymerization of halogenated monomers according to any one of Claims 9 to 11, **characterized in that** the water-insoluble organic solvent is chosen from dialkyl carbonates in which the alkyl groups comprise at most five carbon atoms.

13. Process for the preparation of halogenated polymers by aqueous suspension polymerization of halogenated monomers according to any one of Claims 9 to 12, **characterized in that** the water-insoluble organic solvent is diethyl carbonate.

14. Process for the preparation of halogenated polymers by aqueous suspension polymerization of halogenated monomers according to any one of Claims 9 to 13, **characterized in that** the dialkyl peroxydicarbonate is diethyl peroxydicarbonate.

## Patentansprüche

1. Verfahren zur Herstellung einer organischen Dialkylperoxydicarbonatlösung, **dadurch gekennzeichnet, dass** man in Wasser ein Alkylhalogenformiat in geeigneten Mengen mit einem anorganischen Peroxid zur Reaktion bringt und man das erhaltene Dialkylperoxydicarbonat durch Extraktion mit einem in Wasser unlöslichen organischen Lösungsmittel, das ausgewählt ist unter den Dialkylcarbonaten, abtrennt, um eine Dialkylperoxydicarbonatlösung in diesem Lösungsmittel zu erhalten.

2. Verfahren zur Herstellung einer organischen Dialkylperoxydicarbonatlösung gemäß Anspruch 1, **dadurch gekennzeichnet, dass** das in Wasser unlösliche organische Lösungsmittel unter den Dialkylcarbonaten ausgewählt ist, deren Alkylgruppen höchstens fünf Kohlenstoffatome enthalten.

3. Verfahren zur Herstellung einer organischen Dialkylperoxydicarbonatlösung gemäß einem der Ansprüche 1 bis 2, **dadurch gekennzeichnet, dass** das in Wasser unlösliche organische Lösungsmittel Diethylcarbonat ist.

4. Verfahren zur Herstellung einer organischen Dialkylperoxydicarbonatlösung gemäß einem der Ansprüche 1 bis 3, **dadurch gekennzeichnet, dass** das Dialkylperoxydicarbonat Diethylperoxydicarbonat ist.

5. Organische Dialkylperoxydicarbonatlösung in einem in Wasser unlöslichen organischen Lösungsmittel, **dadurch gekennzeichnet, dass** dieses Lösungsmittel unter den Dialkylcarbonaten ausgewählt ist.

6. Organische Dialkylperoxydicarbonatlösung gemäß Anspruch 5, **dadurch gekennzeichnet, dass** das in Wasser unlösliche organische Lösungsmittel unter den Dialkylcarbonaten ausgewählt ist, deren Alkylgruppen höchstens fünf Kohlenstoffatome enthalten.

7. Organische Dialkylperoxydicarbonatlösung gemäß einem der Ansprüche 5 bis 6, **dadurch gekennzeichnet, dass** das in Wasser unlösliche organische Lösungsmittel Diethylcarbonat ist.

8. Organische Dialkylperoxydicarbonatlösung gemäß einem der Ansprüche 5 bis 7, **dadurch gekennzeichnet, dass** das Dialkylperoxydicarbonat Diethylperoxydicarbonat ist.

9. Verfahren zur Herstellung von halogenierten Polymeren durch Polymerisation in wässriger Suspension von halogenierten Monomeren unter Beteiligung von Dialkylperoxydicarbonat, **dadurch gekennzeichnet, dass** das Dialkylperoxydicarbonat bei der Polymerisation in der Form einer organischen Lösung in einem in Wasser unlöslichen organischen Lösungsmittel, das unter den Dialkylcarbonaten ausgewählt ist, eingesetzt wird.

10. Verfahren zur Herstellung von halogenierten Polymeren durch Polymerisation in wässriger Suspension von halogenierten Monomeren gemäß Anspruch 9, **dadurch gekennzeichnet, dass** die halogenierten Polymere Fluor enthaltende Polymere sind.

11. Verfahren zur Herstellung von halogenierten Polymeren durch Polymerisation in wässriger Suspension von halogenierten Mononneren gemäß einem der Ansprüche 9 bis 10, **dadurch gekennzeichnet, dass** die halogenierten Polymere Vinylidenfluoridpolymere sind.

12. Verfahren zur Herstellung von halogenierten Polymeren durch Polymerisation in wässriger Suspension von halogenierten Monomeren gemäß einem der Ansprüche 9 bis 11, **dadurch gekennzeichnet, dass** das in Wasser unlösliche organische Lösungsmittel unter den Dialkylcarbonaten ausgewählt ist, deren Alkylgruppen höchstens fünf Kohlenstoffatome enthalten.

13. Verfahren zur Herstellung von halogenierten Polymeren durch Polymerisation in wässriger Suspension von halogenierten Monomeren gemäß einem der Ansprüche 9 bis 12, **dadurch gekennzeichnet, dass** das in Wasser unlösliche organische Lösungsmittel Diethylcarbonat ist.

14. Verfahren zur Herstellung von halogenierten Polymeren durch Polymerisation in wässriger Suspension von halogenierten Monomeren gemäß einem der Ansprüche 9 bis 13, **dadurch gekennzeichnet, dass** das Dialkylperoxydicarbonat Diethylperoxydicarbonat ist.
